# FASCICULE DE BREVET EUROPEEN

(11) **EP 0 664 960 B1**
(45) Date de publication et mention de la délivrance du brevet: **14.10.1998**
(21) Numéro de dépôt: 95400199.6
(22) Date de dépôt: 31.01.1995
(51) Int. Cl.: A23G 3/30, A23L 1/236, C07H 15/04

(54) **Composition de chewing-gum présentant une qualité organoleptique améliorée et procédé permettant de préparer un tel chewing-gum**
Kaugummi-Zusammensetzung mit verbesserten organoleptischen Eigenschaften und Verfahren zur Herstellung derselben
Chewing gum composition with improved organoleptic properties and process for preparing the same

(30) Priorité: 01.02.1994 FR 9401082
(43) Date de publication de la demande: 02.08.1995
(73) Titulaire: Roquette Frères, 62136 Lestrem (FR)
(72) Inventeur: Ribadeau-Dumas, Guillaume, F-59139 Lambersart (FR); Bouvier, Frédéric, F-59000 Lille (FR); Serpelloni, Michel, F-62660 Beuvry les Bethune (FR)
(74) Mandataire: Boulinguiez, Didier

(56) Documents cités:
- EP-A- 0 185 595
- EP-A- 0 220 103
- FR-A- 2 236 005
- DATABASE WPI Week 8541 Derwent Publications Ltd., London, GB; AN 85-253398 & JP-A-60 168 364 (SEKISUI CHEM. IND. CO. LTD.) , 31 Août 1985
- DATABASE WPI Week 9413 Derwent Publications Ltd., London, GB; AN 94-105984 & JP-A-06 054 649 (TOWA KASEI KOGYO KK) , 1 Mars 1994

## Description

La présente invention concerne une composition de chewing-gum présentant une qualité organoleptique améliorée. Elle concerne également un procédé de préparation d'une telle composition par emploi d'une poudre de maltitol présentant des caractéristiques très particulières.

La plupart des chewing-gums, qu'ils soient avec ou sans sucre, de type bubble-gum ou non, dragéifiés ou non, comprennent essentiellement une gomme de base insoluble dans l'eau, des agents sucrants hydrosolubles apportés sous forme liquide ou pulvérulente et des arômes. Ils comprennent souvent d'autres ingrédients tels que des colorants, des émulsifiants, des plastifiants, des édulcorants intenses, de l'eau, etc...

Lorsqu'ils sont formulés sans sucre, les chewing-gums du marché comprennent dans la très grande majorité des cas, du sorbitol apporté sous forme pulvérulente. Le diamètre moyen des particules que contiennent les poudres de sorbitol utilisées est très généralement compris entre 100 et 200 microns.

Il existe pour les chewing-gums, et en particulier pour les chewing-gums sans sucre, un défaut constamment ressenti par les consommateurs et non encore très bien résolu par les formulateurs, bien que celui-ci ait fait et fasse toujours l'objet de nombreuses recherches visant à le corriger. Ce défaut, qui est souvent accompagné d'autres défauts d'ordre organoleptique, a trait au manque d'impact des arômes dans les chewing-gums et à leur faible durée de persistance au cours de la mastication. Ainsi, les sensations rétro-olfactives que sont censés procurer les arômes présents dans un chewing-gum, sont généralement insuffisamment intenses durant les premières minutes de mastication et disparaissent le plus souvent très rapidement dans les minutes suivantes. Pourtant, les chewing-gums jugés alors sans goût, contiennent bien souvent encore 60 à 80 % de la quantité d'arome introduite lors de leur formulation.

Plusieurs méthodes ont été décrites dans la littérature afin de résoudre le problème exposé ci-dessus.

Un premier groupe de méthodes part du constat que les arômes pour chewing-gum sont soit trop faiblement solubles dans l'eau et dans ce cas alors fortement liés à la gomme de base du chewing-gum, soit trop fortement solubles dans l'eau et dans ce cas insuffisamment retenus par les constituants du chewing-gum.

Dans le premier cas, qui apparaît être le plus courant, l'arôme, compte tenu de sa faible solubilité dans l'eau et la salive, manque d'impact et est trop faiblement perçu durant les premières minutes de mastication ce qui rend naturellement difficile son identification. Par contre, il est lentement extrait, et ceci de façon continuelle par la salive, ce qui confère habituellement au chewing-gum une certaine persistance de sa flaveur par la suite.

Dans le second cas, l'arôme trop peu lié, est extrait rapidement du chewing-gum, souvent plus rapidement encore que les agents sucrants hydrosolubles. L'arôme présente dans ce cas un excellent impact en bouche mais une persistance particulièrement insuffisante.

Ainsi, le premier groupe de méthodes vise à corriger les défauts de ces deux types d'arômes en modifiant autant que possible, et très directement, leurs coefficients de partage entre d'une part la gomme de base et d'autre part la phase aqueuse ou la salive, de façon à conférer une sensation rétro-olfactive à la fois forte dès la mise en bouche et intense et continuelle par la suite. A ce titre, il est important de rappeler que pour être jugé de bonne qualité par les consommateurs, un chewing-gum doit conserver sa flaveur pendant 10 à 30 minutes, temps correspondant aux temps ordinaires minimum et maximum de mastication.

On connaît en premier lieu parmi ce groupe, les méthodes d'encapsulation des arômes ou de piégeage de ceux-ci dans une matrice. Celle-ci peut être hydrophile auquel cas elle permet un meilleur relargage de l'arôme. Ceci est le cas par exemple dans les procédés décrits dans les brevets et demandes de brevet US 4.122.195, WO 91/00692, US 5.004.595, US 4.695.463, US 3.761.286, EP 102.237, EP 528.466, et US 5.165.943 qui revendiquent respectivement l'emploi pour la création de ces matrices de dextrines, de gomme arabique, de gélatine et d'hydrates de carbone, d'hydrocolloïdes ou de polysaccharides ou de polyols, d'alginates et de carraghénates, d'hydroxyéthyl acrylates, de zéïne, de cellules mirobiennes, de sorbitol et de cyclodextrines.

La matrice protectrice d'encapsulation peut être également hydrophobe comme préconisé par exemple dans les brevets et demandes de brevet EP 401.954, WO 84/03201, US 3.826.847, WO 92/05682 et US 4.590.075 décrivant respectivement l'intérêt d'utiliser des collophanes, des laques ou des cires, de l'acétate de polyvinyle, un mélange de styrène butadiène, de l'octényl succinate d'amidon et du dioxyde de silicone, des gommes synthétiques ou naturelles.

Les inconvénients majeurs de ces techniques se situent au niveau de leur coût élevé, de leur mise en pratique difficile et du risque de ne restituer qu'imparfaitement l'ensemble des notes d'un arôme. Ce risque provient du fait que certaines de ces notes, contrairement à d'autres, peuvent en effet être masquées en raison de leur rétention excessive dans la matrice, ce qui a pour conséquence dommageable de détruire alors l'équilibre original de l'arôme.

En second lieu, parmi ce premier groupe de méthodes, on connaît également des méthodes plus radicales, visant à modifier l'arôme de sorte qu'il devienne plus adapté au besoin spécifique des chewing-gums. On peut citer à ce titre le brevet EP 427.505 préconisant comme solution, l'emploi d'huiles essentielles naturelles déterpénées. Dans cet esprit également, les producteurs d'arômes proposent aujourd'hui d'utiliser des arômes spécialement élaborés pour les chewing-gums sans sucre qui sont d'un prix plus élevé que ceux employés dans les formulations classiques.

On connaît aussi des méthodes intermédiaires, entre les deux techniques proposées plus haut, qui consistent à associer à un arôme liquide adapté, un arôme encapsulé de sorte à obtenir à la fois un impact important et une persistance aromatique élevée. A titre d'exemple, on poura se référer aux brevets EP 265.386 et US 4.001.438. Ces techniques relèvent d'une grande complexité et les inconvénients cités plus haut pour les arômes encapsulés sont généralement retrouvés.

Un deuxième groupe de méthodes exploite la capacité des gommes de base à fixer les arômes. Cette propriété peut être éventuellement ajustée, de façon à créer au sein du chewing-gum un véritable réservoir d'arôme. Cette solution est préconisée par exemple dans les brevets US 4.915.958, US 4.808.418 et US 5.110.608, qui revendiquent des chewing-gums présentant des propriétés organoleptiques améliorées par l'emploi de concentrations élevées en gomme de base. Celle-ci doit alors avoir des propriétés mécaniques et une composition différente de celle ordinairement utilisée de sorte que la mastication de tels chewing-gums soit encore possible. Pour obtenir un résultat satisfaisant, il convient dans ce cas également d'augmenter de façon non négligeable les teneurs en arôme des chewing-gums ce qui n'est pas sans conséquence sur leur prix de revient.

Une autre solution consiste à modifier la composition de la gomme de base. On connaît par exemple la demande de brevet WO 93/17577 dans laquelle il est préconisé pour obtenir le résultat recherché, d'éliminer les cires d'ordinaire employées. Il est impératif alors de reformuler totalement les chewing gums afin qu'ils présentent une liaison correcte et une bonne plasticité.

Un troisième groupe de méthodes consiste à ajouter aux chewing-gums, à de faibles concentrations, des substances particulières de façon à corriger les défauts d'impact et de persistance des arômes.

Il a été proposé dans les brevets et demandes de brevet US 4.752.481, WO 90/04926 et EP 202.885, d'utiliser des émulsifiants dont la balance lipophile-hydrophile est soit inférieure ou soit supérieure à 7, ou encore de la lécithine de soja.

D'autres auteurs ont préconisé l'emploi d'exhausteurs d'arômes tels que les sels ou encore le sel encapsulé, la poudre de cacao, le glutamate de sodium, le méthyl salicylate, les dérivés de limonène. L'emploi de tels produits est par exemple justifié dans les brevets et demandes de brevet WO 90/12512, US 4.889.727, US 3.903.305, US 4.948.595 et US 4.157.401.

Bien d'autres exhausteurs ont été décrits. Il s'agit en particulier d'acides alimentaires et d'édulcorants intenses. Concernant ces derniers, un grand nombre de brevets revendiquent ou décrivent l'intérêt d'utiliser par exemple les cyclamates, l'aspartame, la saccharine, l'alitame, le sucralose, sous forme libre ou encapsulée, pour améliorer le goût des chewing-gums, notamment ceux formulés sans ajoût de sucre. Il semble que l'on recherche alors davantage à augmenter l'intensité et la persistance de la saveur sucrée qu'à améliorer les sensations d'ordre aromatique. On peut faire également appel à d'éventuelles synergies de pouvoir sucrant entre deux différents édulcorants intenses, ou entre un édulcorant intense et un agent édulcorant de masse, ou encore entre deux différents agents édulcorants de masse. On peut citer à titre d'exemple les brevets US 4.986.991 et EP 366.251 relatifs respectivement à une composition synergique entre le sucralose et l'aspartame et à une composition synergique à base de sucralose et de maltitol, utiles à la formulation de chewing-gums. On peut aussi citer le brevet EP 453.402 revendiquant l'emploi d'un mélange synergique comprenant du sucre et du xylitol pour obtenir des chewing-gums de meilleur goût, ou encore le brevet US 5.017.400 relatif à une composition synergique à base de xylitol et de maltitol utilisable par exemple dans la préparation de chewing-gums ayant un goût sucré plus intense.

Les méthodes constituant ce troisième groupe et revendiquant l'emploi de substances particulières, à de faibles concentrations afin de réhausser la perception des arômes de chewing-gums, ne sont pas sans défaut. Elles génèrent parfois des goûts étrangers ou artificiels, ou détruisent l'harmonie que constitue en soi un arôme, masquant certaines notes et rendant plus crues et plus exacerbées certaines autres. L'équilibre originel de l'arôme ne peut être retrouvé alors, que par correction des quantités des différents constituants de l'arôme, lesquels sont en général en nombre très élevé notamment dans le cas des arômes naturels. On comprend la difficulté de réaliser une telle correction.

Un quatrième groupe de méthodes permettant de modifier le goût d'un chewing-gum, et tout au moins, son profil sucré, consiste à ajuster la granulométrie des agents sucrants hydrosolubles apportés sous forme pulvérulente et employés dans la formulation d'un chewing-gum.

On connaît en particulier le brevet EP 427.541 revendiquant l'utilisation de saccharose comportant plus de 60 % de particules de diamètre inférieure à 325 mesh (45 microns) en vue d'augmenter la sensation sucrée dans les chewing-gums. Il est préconisé dans le même temps, pour améliorer l'aromatisation, d'augmenter de 25 % au moins la teneur en gomme de base par rapport aux formulations usuelles et de procéder de même en ce qui concerne la teneur en arôme. Ceci a malheureusement, comme signalé plus haut, des conséquences dommageables sur le coût de revient, mais aussi sur l'aptitude du chewing gum à être aisément mâché.

On connaît également le brevet US 4.900.563 relatif à un chewing-gum anhydre comprenant du fructose pulvérulent de granulométrie inférieure à 70 mesh (212 microns). Malheureusement, le fructose comme le saccharose est cariogène et ne permet pas par conséquent de préparer, comme il est souvent souhaité, des chewing-gums non cariogènes.

On connaît aussi le brevet US 4.803.083 relatif à l'utilisation de deux poudres de sorbitol de granulométries différentes pour préparer des chewing-gums sans sucre. Il s'agit surtout dans ce cas d'améliorer les conditions de fabrication et la texture des produits fabriqués. Bien que l'on se soucie plus des différences existant entre les granulométries de deux poudres que des granulométries elles-mêmes, il convient de signaler qu'apparemment l'une d'entre elles présente un diamètre moyen voisin de 75 microns et la seconde un diamètre moyen préféré bien supérieur à cette valeur, plutôt proche de 200 microns si l'on se réfère à la description. Ainsi des chewing gums non cariogènes peuvent être préparés, mais comme on le verra par la suite, la granulométrie conseillée içi pour le sorbitol, n'est pas celle qu'il convient de retenir pour un maltitol pulvérulent de haute pureté.

La Société demanderesse vient de constater, de façon surprenante et inattendue, que l'on pouvait améliorer la qualité organoleptique d'un chewing-gum, et notamment améliorer le goût et l'aromatisation en termes d'impact et de durée, en lui faisant comporter, en tant que phase pulvérulente, du maltitol ayant une pureté en maltitol supérieure à 95 % et une granulométrie telle que 50 % des particules de maltitol au sein du chewing gum soient de taille inférieure à 90 microns.

Dans ces conditions, on observe une excellente restitution de l'arôme, sans déformation des notes aromatiques de celui-ci. Il n'est donc pas besoin d'ajuster nécessairement la composition de l'arôme dans la mesure où celui-ci convient déjà pour formuler un chewing gum classique, c'est à dire un chewing gum contenant majoritairement du saccharose.

On convient d'appeler, dans la présente invention, "note aromatique" la sensation rétro-olfactive attribuable spécifiquement à un composé d'un arôme considéré isolément ou en relation avec d'autres molécules constitutives de l'arôme.

Par "amélioration de la qualité organoleptique", on entendra, dans la présente invention, l'amélioration de l'ensemble des facteurs sensoriels que sont le goût, l'aromatisation et la texture.

La Société demanderesse a en effet vérifié que lorsque la pureté de la poudre, valeur que l'on peut aisément déterminer par chromatographie, est de l'ordre de 83 % à 94 %, on obtenait alors, à formulation identique, des chewing-gums excessivement durs et difficilement masticables. Pour corriger la texture, il s'avère alors indispensable d'augmenter la teneur en gomme de base et d'augmenter parallèlement la teneur en arôme. Une autre solution consiste à choisir une gomme de base plus souple. Mais ceci conduit dans tous les cas à un enchérissement de la formulation, sans bénéfice d'ordre organoleptique, contrairement à ce qu'on l'on aurait pu penser en se conformant en particulier aux indications du brevet EP 427.541 cité plus haut. De plus, lorsque la pureté du maltitol est faible, la Société demanderesse a constaté que, sans doute en raison de leur cristallinité alors nécessairement basse, les particules de maltitol se solubilisent trop rapidement et la saveur sucrée n'accompagne alors pas l'arôme de façon correcte.

Sans vouloir se référer à une quelconque théorie, lorsque l'on cherche à obtenir une aromatisation améliorée en terme d'impact et de durée, il semble, comme la demanderesse vient de le constater, qu'à chaque édulcorant pour chewing-gum et qu'à chaque niveau de pureté pour un édulcorant choisi, corresponde une granulométrie idéale, le fructose devant être semble-t-il plus grossier que le saccharose, le maltitol plus fin par exemple que le sorbitol, et le maltitol de haute pureté plus fin que le maltitol de basse pureté. Dans le cas d'un maltitol de pureté supérieure à 95 %, la granulométrie idéale au sein d'un chewing-gum est celle qui est spécifiée plus haut.

Un grand nombre de documents enseignent la possibilité d'utiliser du maltitol dans la formulation d'un chewing-gum mais aucun n'envisage de faire comporter, en tant que phase pulvérulente de celui-ci, une poudre de maltitol de haute pureté et de granulométrie adaptée.

Les demandes de brevet et brevets WO 88/08671, US 4.217.368, EP 302.023, EP 425.115, US 5.110.608 et US 5.075.118 par exemple enseignent la possibilité de choisir, parmi plusieurs polyols ou plusieurs édulcorants, le maltitol pour formuler un chewing-gum. L'état physique de ce maltitol n'est pas précisé, ou n'est pas jugé d'une importance capitale. Il peut donc s'agir aussi bien d'un sirop que d'une poudre.

Dans la demande de brevet WO 85/01862 sont donnés des exemples d'utilisation d'une poudre ou d'un sirop de maltose hydrogéné pour protéger et encapsuler l'aspartame destiné notamment à la préparation de chewing-gums non cariogènes. L'état physique du maltose hydrogéné ainsi que sa pureté paraissent sans importance. La granulométrie de la poudre d'aspartame encapsulé dans du maltose hydrogéné, est en revanche comprise entre 40 et 140 mesh, c'est à dire entre 106 à 425 microns, ce qui est loin d'être la granulométrie idéale, comme l'a constaté la demanderesse, pour une poudre de maltitol de haute pureté.

Les brevets JP 74.32067, JP 77.27702 et la demande de brevet JP 03.133.341 exemplifient des procédés de préparation de chewing-gums comportant du maltitol associé à du sucre, du lactose, du dextrose, des sirops de glucose, du mannitol, du sorbitol ou du miel. L'état physique et la pureté du maltitol employé ne sont jamais précisés.

Le document "Chewing-gum sans sucre à base de maltitol" de T. Maruyama et al., publié dans Shokuhin Kogyo (1984), 27, n° 24, p. 73-80 et le brevet EP 325.090 enseignent tous deux la possibilité d'employer du maltitol en remplacement du sorbitol pour préparer des chewing-gums non cariogènes et très faiblement hygroscopiques. Bien que l'état physique du maltitol ne soit pas clairement spécifié, on peut penser qu'il convient de l'utiliser sous forme pulvérulente. Il n'est par contre nullement indiqué de choisir de préférence une granulométrie ou une pureté particulière pour le maltitol.

D'autres documents enseignent plus spécifiquement la possibilité d'utiliser une poudre de maltitol dont la pureté est précisée.

Le document "Evaluation of maltitol in eight food products : comparaison with sucrose, fructose and sorbitol" de R.L. De Fielliettaz Goethart et al. publié dans Basic Studies in Food Science (1983), vol. 2, p. 8-9, fait état de travaux portant sur l'emploi, dans la formulation de chewing-gums, d'une poudre cristalline contenant 88 % de maltitol. Il apparaît que le maltitol a un pouvoir sucrant supérieur à celui du sorbitol et inférieur à celui du sucre. Les propriétés technologiques du maltitol sont estimées tout à fait comparables à celles du sorbitol. Y. FABRY, dans "Malbit^{R} and its application in the food industry" in "Developments in Sweeteners", vol. 3 (1987), p. 83-108, dévoile également une formulation de chewing-gum contenant la même poudre de maltitol MALBIT^{R}, qui présente, comme il le rappelle, une pureté maximale en maltitol de 90 % et une granulométrie voisine de 20 mesh (850 microns) ou de 50 mesh (300 microns).

Enfin, on connaît trois autres documents décrivant des chewing-gums contenant du maltitol d'une pureté pouvant atteindre 99 %.

Le brevet FR 2.499.576 concerne ainsi, à l'exemple 12, un chewing-gum comprenant 30 parties de cristaux anhydres de maltitol de pureté voisine de 99,2 %. La granulométrie de cette poudre de maltitol n'est pas précisée.

Le brevet EP 185.595, quant à lui, décrit à l'exemple 6 un chewing-gum comprenant un maltitol cristallisé dans l'eau, d'une pureté de 94,8 % et dont 65 % des particules ont une taille supérieure à 160 microns.

Le troisième document est le brevet US 5.017.400 qui, comme signalé plus haut, a trait à une composition synergique à base de xylitol et de maltitol. Ce dernier peut être, comme indiqué à l'exemple 1, une poudre cristallisée, broyée et d'une pureté de 99 %. La granulométrie de cette poudre n'est pas spécifiée.

En définitive, aucun des documents ci-dessus n'enseigne, ou ne laisse même entrevoir, l'intérêt de faire comporter en tant que phase pulvérulente d'un chewing-gum, une poudre de maltitol de haute pureté et de granulométrie adaptée afin d'obtenir le résultat recherché dans la présente invention.

L'invention se rapporte donc à une nouvelle composition de chewing-gum comportant essentiellement une gomme de base, un arôme et du maltitol pulvérulent, caractérisée en ce que ledit maltitol présente une pureté en maltitol supérieure à 95 % et une distribution granulométrique telle qu'au moins 50 % en nombre des particules de maltitol présentes au sein de la composition soient de taille inférieure à 90 microns.

La composition présente ainsi une qualité organoleptique améliorée, c'est à dire un meilleur goût et une meilleure aromatisation en termes d'impact et de durée mais aussi une excellente texture, tendre et stable à la mastication et au cours du temps. L'invention se rapporte donc également à une composition de chewing-gum améliorée du point de vue de sa qualité organoleptique comprenant :
- une gomme de base,
- une charge édulcorante comprenant du maltitol pulvérulent, lequel présente une pureté en maltitol supérieure à 95 % et une distribution granulométrique telle qu'au moins 50 % en nombre des particules de maltitol présentes au sein de la composition soient de taille inférieure à 90 microns,
- éventuellement d'autres agents sucrants,
- éventuellement un agent de liaison,
- un agent aromatisant.

La distribution granulométrique du maltitol pulvérulent est, comme indiqué plus haut, d'une grande importance sur l'effet recherché. Elle est de préférence, au sein de la composition de chewing-gum, telle qu'au moins 50 % en nombre des particules de maltitol soient de taille inférieure à 75 microns. Lorsque le maltitol pulvérulent est encore plus finement divisé au sein du chewing-gum, c'est à dire lorsque plus de la moitié des particules ont une taille inférieure à 60 microns, l'effet recherché est encore meilleur. La Société demanderesse a constaté que l'idéal est d'obtenir au sein du chewing-gum au moins 50 % des particules de taille inférieure à 40 microns.

Cette distribution granulométrique du maltitol pulvérulent au sein du chewing-gum se mesure aisément en microscopie photonique en contraste interférentiel sur une coupe de 20 microns d'épaisseur du chewing-gum préalablement congelé à -20°C.

La granulométrie du maltitol pulvérulent donnée ci-dessus est celle, comme l'a constaté la demanderesse, qu'il convient de choisir pour une poudre de maltitol de très haute pureté. L'effet recherché est alors maximum et la texture reste masticable avec des gommes de base ordinaires. On n'est pas alors dans l'obligation, contrairement à ce qu'il convient de faire pour une poudre de maltitol de basse pureté et à ce qui est enseigné en particulier dans le brevet EP 427.541, d'augmenter les quantités de gomme de base et parallèlement les quantités d'arôme. Le choix d'un maltitol pulvérulent de haute pureté se justifie également, comme l'a constaté la Société demanderesse, pour des raisons d'ordre technologique, liées à la plus grande facilité de broyage, à la moindre production de poussières et à la plus grande aisance de stockage avec un risque moindre de mottage, ceci par rapport à une poudre de maltitol de pureté inférieure à 95 %.

C'est ainsi qu'on préfère faire comporter à la composition de chewing-gum conforme à l'invention du maltitol pulvérulent d'une pureté supérieure à 98 %, de préférence supérieure à 99 % et plus préférentiellement supérieure à 99,5 %.

La mesure de pureté du maltitol pulvérulent distribué au sein du chewing-gum, peut être réalisée par chromatographie en phase gazeuze après silylation. La quantification se fait alors par la méthode de l'étalonnage interne, en employant une poudre de maltitol de pureté connue.

Les particules du maltitol ont une haute cristallinité. Leur enthalpie de fusion dépasse 125 joules par gramme et d'ordinaire dépasse 140 joules par gramme, voire 160 joules par gramme. De plus, ces particules ont de préférence un point de fusion mesuré par analyse calorimétrique différentielle supérieur à 145°C et de préférence supérieur à 147°C.

Il est à noter que plus le maltitol pulvérulent est pur, plus il a été constaté qu'il était intéressant de faire comporter à la composition de chewing-gum des particules très fines. Il semble donc qu'il y ait une relation immédiate entre pureté et granulométrie pour obtenir un goût amélioré et une texture masticable restant stable.

Le maltitol pulvérulent possédant au sein de la composition de chewing-gum les caractéristiques énoncées ci-dessus, peut représenter environ 2 % à environ 85 % de la composition. L'effet est d'autant meilleur que sa concentration est élevée. On préfère, pour cela, retenir des quantités comprises entre 5 % et 75 %, et de préférence strictement comprises entre 10 % et 75 % de la composition.

La gomme de base constitutive de la composition de chewing-gum est de préférence ordinaire et semblable à celles qui sont couramment utilisées. Elle peut représenter, selon qu'il s'agit d'une tablette à mâcher, d'un bubble-gum, d'un centre à dragéifier, ou d'un chewing-gum hypocalorique, environ 15 % à environ 70 % de la composition conforme à l'invention. Sa nature sera également adaptée au type de chewing-gum fabriqué. Elle pourra comprendre des élastomères synthétiques et/ou naturels comme le polyisoprène, l'acétate de polyvinyle, le polyisobutylène, des latex, des résines comme les résines terpéniques, les esters et les alcools de polyvinyl, des matières grasses ou des cires comme par exemple la lanoline, les huiles végétales partiellement hydrogénées ou non, les acides gras, les esters partiels de glycérol, la paraffine, les cires microcristallines, des agents de charge comme du talc, le carbonate de calcium, des plastifiants d'élastomères comme le triacétate de glycérol, le monostéarate de glycérol, les dérivés de collophanes, des émulsifiants comme la lécithine, les esters de sorbitol, des colorants ou des agents de blanchiment, des antioxydants, des agents anticollants comme le mannitol.

De préférence, la teneur en gomme de base de la composition conforme à l'invention est comprise entre 15 % et 40 %. Cette teneur est, dans la majorité des cas, comprise entre 18 % et 28 %, c'est à dire légèrement inférieure aux teneurs habituelles des chewing-gums sans sucre du marché formulés avec du sorbitol en tant qu'édulcorant de masse pulvérulent prépondérant.

La composition de chewing-gum conforme à l'invention contient aussi un agent aromatisant. Cet agent peut comprendre des composés naturels et/ou de synthèse. Il peut s'agir en particulier d'arômes de menthe, de cannelle, d'orange, de citron, de limette ou d'arômes correspondant à d'autres fruits ou plantes tels par exemple les arômes de pomme, de fraise, de banane, de cerise ou de mélanges de fruits.

L'agent aromatisant est employé en une quantité appropriée aisément déterminable par l'homme du métier par de simples essais de routine, en considérant la nature de la gomme de base, la quantité de gomme de base, le type de chewing-gum et les caractéristiques de cet agent aromatisant. D'ordinaire, il sera employé à un taux compris entre environ 0,2 % et environ 3 %. De préférence, on retiendra, notamment pour les agents aromatisants hydrophobes, des quantités suffisantes pour plastifier la gomme de base sans que le ramollissement de celle-ci soit excessif. Pour cela, on retiendra plutôt une teneur en agent aromatisant comprise entre 0,5 % et 1,8 %, l'idéal étant de retenir une teneur comprise entre 0,8 % et 1,5 %.

Le dosage en agent aromatisant dépendra également de la richesse de celui-ci en composés d'arômes, c'est à dire en composés ayant réellement un effet rétro-olfactif. De plus, ce dosage variera avec la nature physique de l'agent aromatisant. Par exemple, pour une forme encapsulée, le dosage sera d'ordinaire plus faible.

L'agent aromatisant peut se présenter sous forme d'un produit unique ou sous deux ou plusieurs formes physiques différentes comprenant essentiellement les mêmes composés d'arôme.

On peut employer également plusieurs agents aromatisants de natures différentes et d'états physiques identiques ou différents.

La composition de chewing-gum selon l'invention peut comprendre un agent de liaison, dans une concentration de 0,1 % à 30 %. Celui-ci peut être choisi de préférence parmi l'eau, la glycérine, les sirops de mono, di, oligo ou polysaccharides, hydrogénés ou non, et les sirops d'agents de charge hypocaloriques et les mélanges quelconques d'entre eux.

L'eau du chewing gum peut être apportée sous forme d'eau libre ou par d'autres constituants.

Les sirops de mono, di, oligo ou polysaccharides peuvent être par exemple des sirops de xylitol, de sorbitol, de maltitol, de lactitol, d'isomaltulose, d'isomaltulose hydrogéné, d'érythrose, d'érythritol, des sirops, de préférence hydrogénés, issus de l'hydrolyse d'amidons ou d'inulines, contenant des oligosaccharides et/ou des polysaccharides. Quant aux sirops d'agents de charge hypocaloriques, on préfère retenir en particulier les sirops de polydextrose, de polyglucose, de dextrine.

La composition selon l'invention peut contenir également des agents édulcorants additionnels sous forme sèche ou liquide. Il peut s'agir d'édulcorants massiques et/ou d'édulcorants intenses ayant un pouvoir sucrant au moins dix fois supérieur à celui du saccharose. Parmi les édulcorants massiques, il peut en particulier s'avérer intéressant d'utiliser du mannitol pour prolonger la saveur sucrée, de l'érythritol et du xylitol pour apporter une certaine fraîcheur, du sorbitol pulvérulent ou une poudre de maltitol de pureté inférieure à 95 % pour ajuster la texture et rendre celle-ci plus ferme. Lorsqu'une poudre de maltitol d'une pureté comprise entre 82 et 94 % est ajoutée, par exemple dans ce but, elle pourra l'être éventuellement en prémélange en toute proportion avec le maltitol pulvérulent d'une pureté supérieure à 95 %, ou bien avec un autre ingrédient de la composition selon l'invention. Cette composition peut également comprendre des édulcorants intenses tels que l'aspartame, l'alitame, l'acésulfame, le sucralose, sous forme libre et/ou encapsulée.

Des acides alimentaires peuvent être également ajoutés dans la composition conforme à l'invention, par exemple en tant qu'exhausteurs, à de faibles teneurs, notamment lorsqu'un arôme fruité est employé.

La présente invention concerne également un procédé permettant d'améliorer le goût et la texture d'un chewing-gum mais permettant aussi d'améliorer la restitution de l'arôme présent dans un chewing gum. Ce procédé s'applique en particulier à un chewing-gum sans sucre. Le procédé consiste à choisir et à utiliser un maltitol pulvérulent particulier, de sorte que le chewing-gum comporte en fin de fabrication des particules de maltitol d'une pureté en maltitol supérieure à 95 % et d'une distribution granulométrique telle qu'au moins 50 % d'entre-elles soient au sein du chewing-gum de taille inférieure à 90 microns, de préférence inférieure à 75 microns et plus préférentiellement inférieure à 60 microns. L'idéal est de faire en sorte que plus de la moitié des particules de maltitol soit inférieure à 40 microns dans le chewing-gum. Le principe du procédé consiste à choisir une composition de maltitol appropriée. Après de nombreux essais, la demanderesse a constaté, de façon surprenante et inattendue, qu'il convenait de retenir et d'employer une composition de granulométrie, de compressibilité et de pureté en maltitol ajustées en fonction du type de chewing-gum à formuler. Cette composition de maltitol n'est pas nécessairement dans tous les cas totalement anhydre.

La Société demanderesse a constaté de façon surprenante qu'il y avait une relation directe entre l'aptitude d'une poudre à donner des comprimés durs, c'est à dire à être compressible, et la taille des particules à retenir pour obtenir un goût amélioré et une texture masticable restant à la fois stable au cours de la mastication et au vieillissement.

En premier lieu, lorsqu'on souhaite préparer un chewing-gum devant posséder une qualité organoleptique améliorée et être de plus quasiment dépourvu de phase liquide, c'est à dire d'eau et de glycérine, on peut choisir d'employer un maltitol pulvérulent faiblement compressible, pouvant posséder alors une granulométrie voisine ou très légèrement plus grossière que celle qu'il convient d'obtenir au sein du chewing-gum. On peut aussi retenir un maltitol pulvérulent hautement compressible. Dans ce cas, il est préférable de retenir une granulométrie nettement supérieure à celle que l'on désire obtenir en final dans le chewing-gum.

Une compressibilité est considérée comme faible dans la présente invention lorsqu'on obtient pour le maltitol pulvérulent retenu une valeur inférieure à 80 N selon la méthode "test A" décrite dans le brevet EP 220.103 dont la demanderesse est titulaire. Une compressibilité est considérée comme élevée dans le cas contraire, c'est à dire lorsque cette valeur dépasse 80 N.

Le test A consiste à mesurer la force, exprimée en Newton, qui est représentative de la compressibilité du maltitol pulvérulent étudié et qui est nécessaire pour provoquer l'écrasement d'un comprimé préparé à partir dudit maltitol, c'est-à-dire pour provoquer l'apparition de lignes de rupture au sein de la masse constitutive de celui-ci, cette force traduisant donc la résistance à l'écrasement du comprimé, qui est cylindrique à faces planes, d'un diamètre de 13 mm, d'une épaisseur de 5 mm et d'un poids de 0,896 g, c'est à dire d'une masse volumique apparente de 1,35 g/ml, ladite force étant exercée contre la surface périphérique du comprimé en direction de l'axe de révolution de celui-ci, au moyen d'une butée mobile s'appliquant contre ladite surface le long d'une génératrice, ledit comprimé étant par ailleurs immobilisé contre une butée fixe appliquée également contre la surface périphérique du comprimé le long d'une génératrice diamétralement opposée à celle contre laquelle s'applique la butée mobile.

Ainsi, lorsque le maltitol pulvérulent est composé de cristaux obtenus par cristallisation dans l'eau, d'une pureté dépassant 95 % et présentant une compressibilité inférieure à 80 N dans le test A et d'ordinaire voisine de 45 N, on peut choisir une granulométrie sensiblement identique à celle que l'on désire obtenir dans le chewing-gum dépourvu de phase liquide, c'est à dire telle qu'au moins 50 % des particules soient de taille inférieure à 90 microns.

En revanche, pour un produit de haute pureté et hautement compressible, tel que celui faisant l'objet de l'invention du brevet EP 220.103, la demanderesse a constaté que l'on pouvait choisir, sans que cela soit obligatoire mais seulement préféré, une granulométrie plus grossière, c'est à dire telle qu'au moins 50 % des particules aient une taille inférieure à 120 microns, voire 150 microns. Au sein du chewing-gum, en procédant de la sorte, on peut obtenir ainsi, en fin de fabrication, la distribution granulométrique idéale pour ce qui concerne le goût et la texture.

On peut également, pour parvenir à ce résultat, retenir un mélange d'un maltitol pulvérulent de haute pureté faiblement compressible et d'une poudre de maltitol hautement compressible et de pureté inférieure à 95 % en maltitol.

En second lieu, lorsqu'on désire préparer un chewing-gum contenant en tant que phase liquide uniquement de la glycérine, c'est à dire quasiment dépourvu d'eau, on peut retenir le principe exposé ci-dessus sauf lorsque le procédé de fabrication du chewing-gum avec glycérine impose, lors du mélange ou de l'extrusion, des conditions de température sévères en présence de la composition du maltitol. On peut en effet dans ce cas choisir, comme l'a constaté la demanderesse, une granulométrie légèrement plus grossière encore que celle qu'il convient de retenir pour un chewing-gum quasiment dépourvu de phase liquide.

Ceci peut s'expliquer par le fait que le maltitol cristallisé de haute pureté est légèrement soluble dans la glycérine anhydre. En effet, sa solubilité à 68°C, à 90°C et à 115°C est respectivement proche de 5 %, de 20 % et de 50 % dans le milieu considéré, ce qui est loin d'être négligeable. Par conséquent, à haute température, le maltitol parait pouvoir se solubiliser dans la glycérine au sein du chewing gum. Ainsi, une granulométrie assez grossière peut être choisie pour formuler un chewing-gum anhydre avec glycérine lorsqu'on travaille à haute température de mélange ou d'extrusion.

En troisième lieu, lorsqu'on souhaite formuler un chewing-gum contenant de l'eau, à raison de 0,1 % à 6 %, on peut choisir une composition de maltitol d'autant plus grossière qu'elle est compressible, que les températures de mélange ou d'extrusion sont élevées et que le chewing-gum comporte des teneurs en eau et en glycérine élevées. Il appartiendra à l'homme du métier, en considérant les règles simples énoncées ci-dessus, de choisir la granulométrie adéquate du maltitol pulvérulent de haute pureté, pour obtenir au sein du chewing-gum une distribution telle qu'au moins 50 % des particules de maltitol soient de taille inférieure à 90 microns.

Il est à noter par ailleurs qu'on doit se soucier, pour déterminer la granulométrie adéquate, du moment choisi au cours du procédé pour introduire le maltitol pulvérulent. En effet, en retenant une poudre excessivement fine et en l'introduisant en tout début de fabrication, une solubilisation trop importante du maltitol se produit généralement, notamment lors d'un travail à température élevée et en présence de quantités d'eau ou de glycérine élevées. En conséquence, la pâte de chewing-gum a dans ce cas tendance à devenir collante, ce qui n'est pas sans poser des problèmes au cours de la fabrication. Par ailleurs, au refroidissement et au stockage, le maltitol solubilisé est alors amené à recristalliser et le chewing-gum pourra contenir des particules grossières et ne plus présenter une aromatisation correcte en terme d'impact et de durée.

Par conséquent, il convient autant que possible d'éviter une solubilisation excessive du maltitol pulvérulent par le choix judicieux d'une granulométrie correcte en fonction des paramètres de fabrication et de la formulation retenue.

Ces trois types de chewing-gum peuvent être fabriqués sans inconvénient en utilisant les procédés et les méthodes connus en soi pour les chewing-gums. Les produits finaux peuvent être tout aussi bien vendus en tablettes à mâcher qu'en coussinets ou boules de gomme dragéifiés ou encore en bubble-gums destinés en particulier aux enfants.

L'invention concerne enfin, en tant que produit nouveau, un maltitol pulvérulent, destiné en particulier à la préparation d'un chewing gum possédant une qualité organoleptique améliorée. Le maltitol pulvérulent conforme à l'invention est particulier en ce sens qu'il possède à la fois une pureté extrêmement élevée et une granulométrie fine bien choisie. Sa pureté en maltitol, que l'on peut quantifier par chromatographie liquide haute performance sur résine échangeuse d'ions mise sous forme calcium, doit être supérieure à 95 % .

La granulométrie du maltitol pulvérulent, qui peut être déterminée par observation au microscope, doit être telle qu'au moins 50 % en nombre des particules soient de taille, exprimée en microns, inférieure à 1,5 fois la valeur de compressibilité dudit maltitol exprimée en Newton (N) et déterminée dans le test A tel que décrit dans le brevet EP 220.103 précité. De plus, le maltitol pulvérulent conforme à l'invention contient de préférence une teneur inférieure à 35 % par rapport au poids du maltitol pulvérulent, en particules de taille inférieure à 10 microns.

La pureté, qui se révèle être un paramètre d'une grande importance en relation avec l'aisance du broyage, la texture et la perception des arômes dans un chewing gum, est de préférence supérieure à 98 %, plus préférentiellement supérieure à 99 % et mieux encore supérieure à 99,5 %.

D'ordinaire, les particules du maltitol pulvérulent ont une enthalpie de fusion supérieure à 140 joules par gramme, voire 160 joules par gramme, et un point de fusion supérieur à 145°C et de préférence supérieur à 147°C.

Concernant la granulométrie, le maltitol pulvérulent comprend de préférence des particules hautement cristallines de granulométrie telle que 50 % en nombre de ces particules soient de taille inférieure, en microns, à 1,2 fois, et plus préférentiellement à 0,8 fois, la valeur de compressibilité obtenue en newton dans le test A précité.

Enfin, la teneur en particules de taille inférieure à 10 microns, lesquelles se révèlent être celles qui créent le plus de poussière dans les ateliers de production et celles susceptibles de se solubiliser le plus rapidement dans la phase liquide d'un chewing gum, est de préférence inférieure à 25 %, plus préférentiellement inférieure à 15 % et mieux encore inférieure à 5 % en poids du maltitol pulvérulent.

L'invention sera mieux comprise à l'aide des exemples qui suivent.

### EXEMPLE 1 : Incidence de la granulométrie

Des compositions de chewing-gum aromatisées, sont préparées selon la formulation de base ci-dessous :

| | |
|---|---|
| Gomme de base DREYCO^{R} (Dreyfus) | 20 % |
| Maltitol en poudre | 63,7 % |
| LYCASIN^{R} 80/55 concentré, commercialisé par la Société demanderesse | 15 % |
| Glycérine | 0,5 % |
| Arôme naturel de menthe (Silesia) | 0,8 % . |

La gomme de base est introduite dans un pétrin-malaxeur possédant deux bras en Z et une double enveloppe, maintenu à une température de 50°C par circulation d'eau dans la double enveloppe. Après 5 minutes environ de malaxage de la gomme de base, environ le tiers du maltitol poudre est ajouté et mélangé intimement à la gomme de base. On procède à un malaxage continuel de 2 minutes, puis on ajoute un deuxième tiers du maltitol en poudre et la quantité de LYCASIN^{R} 80/55 préchauffé à 55°C. Après environ 2 minutes de malaxage supplémentaire, on introduit la quantité de maltitol en poudre restante et la glycérine. Enfin, après 2 minutes de malaxage, on ajoute l'arôme de menthe liquide. On maintient encore le malaxage pendant 1 minute de façon à obtenir une pâte homogène. Celle-ci est ensuite sortie du pétrin, laminée et découpée en sticks de 5 millimètres d'épaisseur.

On prépare, selon cette formulation, cinq compositions de chewing-gum en utilisant les cinq poudres de maltitol suivantes, toutes de pureté supérieure à 95 % et toutes obtenues par cristallisation dans l'eau. Celles-ci présentent une compressiblité voisine de 45 N dans le test A et les caractéristiques suivantes :

| - Poudre A : | |
|---|---|
| . pureté en maltitol (par chromatographie en phase liquide) | 99,6 % |
| . teneur en eau (méthode de Karl Fisher) | 0,27 % |
| . point de fusion | 148°C |

| - Poudre B : | |
|---|---|
| . pureté en maltitol | 99,1 % |
| . teneur en eau | 0,44 % |
| . point de fusion | 149°C |

| - Poudre C : | |
|---|---|
| . pureté en maltitol | 98,4 % |
| . teneur en eau | 0,29 % |
| . point de fusion | 147°C |

| - Poudre D : | |
|---|---|
| . pureté en maltitol | 99,4 % |
| . teneur en eau | 0,26 % |
| . point de fusion | 148,5°C |

| - Poudre E : | |
|---|---|
| . pureté en maltitol | 98,4 % |
| . teneur en eau | 0,32 % |
| . point de fusion | 147°C |

Les distributions granulométriques de ces poudres sont telles que 50 % de particules en poids de maltitol sont de taille voisine de :
- 150 microns pour la poudre A
- 95 microns pour la poudre B
- 75 microns pour la poudre C
- 60 microns pour la poudre D
- 40 microns pour la poudre E

### 1.1) Mesures de dureté

On compare les duretés, pour les cinq compositions de chewing-gum, de sticks de 0,5 centimètre d'épaisseur, agés de 8 jours et stockés à l'abri de l'humidité à 20°C, par pénétrométrie à l'aide d'un appareil de marque INSTRON^{R}.

Les résultats moyens de dureté (valeurs moyennes pour 10 sticks) sont les suivants :

| | |
|---|---|
| Poudre A | 32,0 N |
| Poudre B | 35,3 N |
| Poudre C | 25,6 N |
| Poudre D | 35,9 N |
| Poudre E | 35,4 N |

On constate que les compositions de chewing gum ont des duretés assez proches, sauf dans le cas de l'utilisation de la poudre C où les sticks obtenus sont plus souples.

Pour l'ensemble des compositions, les textures sont jugées bonnes, voire très bonnes.

### 1.2) Evaluation du goût

On demande à un panel de 25 personnes, dans un test en aveugle, de noter de 0 à 4 les cinq compositions de chewing gum par ordre de préférence (note 0 pour le produit jugé de moins bon goût et note 4 pour le produit préféré).

Ces notes sont ensuite rassemblées et interprétées en tenant compte des remarques de chaque dégustateur.

La composition comprenant la poudre A est celle qui obtient le score le plus bas (1,2).

Cette composition se distingue de manière significative des autres compositions testées par la note moyenne attribuée. De plus, la perception de l'arôme, durant les premières secondes de mastication, est jugée pour cette composition trop tardive. Le goût sucré est intense mais éphémère et n'accompagne pas de façon correcte la perception de l'arôme. Ceci n'est pas le cas des compositions à base des poudres B, C, D et E pour lesquelles le goût et l'aromatisation sont grandement améliorés en terme d'impact et de durée.

Les compositions préférées sont celles qui comprennent les poudres C, D et E. Elles obtiennent des scores compris entre 2,9 et 3,8 ; ce qui est remarquable.

### 1.3) Taille des particules au sein des compositions de chewins gum.

Les distributions granulométriques du maltitol pulvérulent au sein des compositions sont évaluées par microscopie photonique en contraste différentiel sur des coupes de chewing-gums préalablement congelés à -20°C. Ces coupes ont une épaisseur de 20 microns environ. On constate que ces distributions granulométriques sont dans tous les cas environ 10 % inférieures à celles des poudres de maltitol utilisées. Ces distributions sont telles qu'au moins 50 % des particules de maltitol sont de taille inférieure à 90 microns et sont donc conformes à l'invention, sauf dans le cas de l'emploi de la poudre A, où les particules sont plus grossières.

### EXEMPLE.2. : Incidence de la pureté en maltitol

On prépare six autres compositions de chewing gum en utilisant la formulation et le mode opératoire donnés à l'exemple 1 mais en faisant comporter à ces compositions les poudres de maltitol suivantes :
- La poudre C
- La poudre E
- Une poudre F présentant :
   . Une compressibilité dans le test A voisine de 55 N
   . Une pureté en maltitol de 83,9 %
   . Une teneur en eau de 1,9 %
   . Une teneur en sorbitol de 4,4 %
   . Un point de fusion de 120°C
   . Une distribution granulométrique telle que 50 % en nombre des particules de maltitol soient de taille voisine de 75 microns.
- Une poudre G présentant :
   . Une compressibilité dans le test A voisine de 175 N
   . Une pureté en maltitol de 92,6 %
   . Une teneur en eau de 1,1 %
   . Une teneur en sorbitol de 1,7 %
   . Un point de fusion de 135°C
   . Une distribution granulométrique telle que 50 % en nombre des particules de maltitol soient de taille voisine de 90 microns
- Une poudre H présentant :
   . Une compressibilité dans le test A voisine de 180 N
   . Une pureté en maltitol de 94,0 %
   . Une teneur en eau de 1,0 %
   . Une teneur en sorbitol de 1,2 %
   . Un point de fusion de 143°C
   . Une distribution granulométrique telle que 50 % en nombre des particules de maltitol soient de taille voisine de 20 microns.
- Une poudre I présentant :
   . Une compressibilité dans le test A voisine de 50 N
   . Une pureté en maltitol de 99,4 %
   . Une teneur en eau de 0,5 %
   . Une teneur en sorbitol de 0,3 %
   . Un point de fusion de 147,6°C
   . Une distribution granulométrique telle que 50 % en nombre des particules de maltitol soient de taille voisine de 65 microns.

Par commodité, on appellera respectivement Composition C, Composition E, Composition F, Composition G, Composition H et Composition I, les compositions de chewing gums obtenues.

Les compositions G et H présentent une mauvaise liaison et une mauvaise homogénéité.

En revanche, les autres compositions de chewing gum apparaissent acceptables en fin de fabrication.

### 2.1) Mesures de dureté

On a obtenu les résultats suivants sur des sticks de 0,5 centimètre d'épaisseur stockés à 20°C à l'abri de l'humidité pendant 1 jour et 8 jours après fabrication.

| | | | |
|---|---|---|---|
| Composition C | à 1 jour | 20,0 N | |
| | | à 8 jours | 25,8 N |
| Composition E | à 1 jour | 24,5 N | |
| | | à 8 jours | 35,4 N |
| Composition F | à 1 jour | 27,7 N | |
| | | à 8 jours | 60,9 N |
| Composition G | à 1 jour | 74,2 N | |
| | | à 8 jours | 91,1 N |
| Composition H | à 1 jour | 100,0 N | |
| | | 8 jours | 91,1 N |
| Composition I | à 1 jour | 31,4 N | |
| | | à 8 jours | 44,4 N |

Un jour après fabrication, les compositions G et H sont excessivement dures. Les textures de ces produits apparaissent incorrectes également 8 jours après fabrication, aussi bien à la mâche que par pénétrométrie.

Ces textures peuvent être améliorées soit en augmentant la teneur en gomme de base, soit en choisissant une gomme de base de plasticité nettement plus élevée.

Les compositions F et I sont relativement dures après un jour.

Ces textures sont jugées acceptables. En revanche, après 8 jours, la composition F devient très difficilement masticable, ce qui n'est pas le cas pour la composition I qui apparait stable dans le temps.

Les compositions C et E sont également stables et conservent une texture correcte et sont aisément masticables.

En définitive, seul l'emploi de poudres de maltitol d'une pureté supérieure à 95 % et de granulométrie correcte convient à la réalisation de compositions de chewing gum stables et demeurant masticables.

### 2.2) Evaluation du goût :

Il apparait que les résultats de dureté obtenus par les dégustateurs du panel de l'exemple 1 sont en total accord avec ceux obtenus instrumentalement et donnés plus haut.

En terme de goût, les compositions C, E et I sont significativement préférées par rapport aux autres compositions, en raison de l'apparition de la flaveur dès les premières dizaines de secondes qui suivent la mise en bouche et en raison de la persistance élevée au cours de la mastication de la saveur sucrée et de l'arôme.

## Revendications

1. Composition de chewing-gum comportant une gomme de base, un arôme et du maltitol pulvérulent, caractérisée en ce que ledit maltitol présente une pureté en maltitol supérieure à 95 % et une distribution granulométrique telle qu'au moins 50 % des particules de maltitol présentes au sein de la composition sont de taille inférieure à 90 microns.

2. Composition de chewing-gum selon la revendication 1, caractérisée en ce que le maltitol présente une distribution granulométrique telle qu'au moins 50 % des particules sont de taille inférieure à 75 microns, de préférence de taille inférieure à 60 microns, et plus préférentiellement encore de taille inférieure à 40 microns.

3. Composition de chewing-gum selon la revendication 1 caractérisée en ce que le maltitol présente une pureté en maltitol supérieure à 98 %, de préférence supérieure à 99 %, et plus préférentiellement encore supérieure à 99,5 %.

4. Composition de chewing-gum selon la revendication 1, caractérisée en ce que le maltitol représente d'environ 2 à environ 85 % de la composition, de préférence de 5 % à 75 % de la composition, et plus préférentiellement encore de 10 % à 75 % de la composition.

5. Composition de chewing-gum selon la revendication 1, dans laquelle l'agent aromatisant représente environ 0,2 % à environ 3 %, de préférence de 0,5 % à 1,8 %, et plus préférentiellement encore de 0,8 % à 1,5 % de la composition.

6. Composition de chewing gum selon la revendication 1, comprenant en outre une poudre de maltitol de pureté inférieure à 95 %.

7. Procédé permettant d'améliorer la qualité organoleptique d'un chewing gum en termes de goût et de texture caractérisé en ce qu'il consiste à utiliser un maltitol pulvérulent ayant une pureté en maltitol supérieure à 95 % et ayant une granulométrie et une compressibilité choisies de sorte qu'au moins 50 % des particules de maltitol présentes au sein du chewing gum sont de taille inférieure à 90 microns.

8. Utilisation pour améliorer la qualité organoleptique d'un chewing gum, d'un maltitol pulvérulent présentant une pureté en maltitol supérieure à 95 % et une granulométrie telle qu'au moins 50 % en nombre des particules sont de taille, exprimée en microns, inférieure à 1,5 fois la valeur de compressibilité dudit maltitol déterminée dans un test A consistant à mesurer la force, exprimée en Newton, nécessaire pour provoquer l'écrasement d'un comprimé préparé à partir dudit maltitol, c'est-à-dire pour provoquer l'apparition de lignes de rupture au sein de la masse constitutive de celui-ci, cette force traduisant donc la résistance à l'écrasement du comprimé qui est cylindrique à faces planes d'un diamètre de 13 mm, d'une épaisseur de 5 mm et d'un poids de 0,896 g, c'est à dire d'une masse volumique apparente de 1,35 g/ml, ladite force étant exercée contre la surface périphérique du comprimé en direction de l'axe de révolution de celui-ci au moyen d'une butée mobile s'appliquant contre ladite surface le long d'une génératrice, ledit comprimé étant par ailleurs immobilisé contre une butée fixe appliquée également contre la surface périphérique du comprimé le long d'une génératrice diamétralement opposée à celle contre laquelle s'applique la butée mobile.

9. Maltitol pulvérulent, utilisable en particulier pour la fabrication d'un chewing gum possédant une qualité organoleptique améliorée, caractérisé en ce que sa pureté en maltitol est supérieure à 95 % et en ce que sa granulométrie est telle qu'au moins 50 % en nombre des particules sont de taille, exprimée en microns, inférieure à 1,5 fois la valeur de compressibilité déterminée dans un test A consistant à mesurer la force, exprimée en Newton, nécessaire pour provoquer l'écrasement d'un comprimé préparé à partir dudit maltitol, c'est-à-dire pour provoquer l'apparition de lignes de rupture au sein de la masse constitutive de celui-ci, cette force traduisant donc la résistance à l'écrasement du comprimé qui est cylindrique à faces planes d'un diamètre de 13 mm, d'une épaisseur de 5 mm et d'un poids de 0,896 g, c'est à dire d'une masse volumique apparente de 1,35 g/ml, ladite force étant exercée contre la surface périphérique du comprimé en direction de l'axe de révolution de celui-ci au moyen d'une butée mobile s'appliquant contre ladite surface le long d'une génératrice, ledit comprimé étant par ailleurs immobilisé contre une butée fixe appliquée également contre la surface périphérique du comprimé le long d'une génératrice diamétralement opposée à celle contre laquelle s'applique la butée mobile.

## Claims

1. Chewing-gum composition composed of a gum base, a flavouring and pulverulent maltitol, characterized in that the said maltitol has a maltitol purity greater than 95% and a particle size distribution such that at least 50% of the maltitol particles present within the composition are smaller than 90 microns in size.

2. Chewing-gum composition according to Claim 1, characterized in that the maltitol has a particle size distribution such that at least 50% of the particles are smaller than 75 microns in size, preferably smaller than 60 microns in size and even more preferably smaller than 40 microns in size.

3. Chewing-gum composition according to Claim 1, characterized in that the maltitol has a maltitol purity greater than 98%, preferably greater than 99% and even more preferably greater than 99.5%.

4. Chewing-gum composition according to Claim 1, characterized in that the maltitol represents from approximately 2 to approximately 85% of the composition, preferably from 5% to 75% of the composition and even more preferably from 10% to 75% of the composition.

5. Chewing-gum composition according to Claim 1, in which the flavouring agent represents approximately 0.2% to approximately 3%, preferably from 0.5% to 1.8% and even sore preferably from 0.8% to 1.5% of the composition.

6. Chewing-gum composition according to Claim 1, additionally comprising a maltitol powder having a purity of less than 95%.

7. Process enabling the organoleptic quality of a chewing-gum to be improved in terms of taste and texture, characterized in that it consists in using a pulverulent maltitol having a maltitol purity greater than 95% and having a particle size and a compressibility which are chosen such that at least 50% of the maltitol particles present within the chewing-gum are smaller than 90 microns in size.

8. Use, in order to improve the organoleptic quality of a chewing-gum, of a pulverulent maltitol having a maltitol purity greater than 95% and a particle size such that at least 50% in number of the particles are smaller in size, expressed in microns, than 1.5 times the compressibility value of the said maltitol, determined in a test A consisting in measuring the force, expressed in newtons, necessary to crush a tablet prepared from the said maltitol, that is to say to cause the appearance of rupture lines within the bulk constituting the latter, this force thus reflecting the crush strength of the tablet, which is cylindrical with plane faces having a diameter of 13 mm, a thickness of 5 mm and a weight of 0.896 g, that is to say having an apparent voluminal mass of 1.35 g/ml, the said force being exerted against the peripheral surface of the tablet in the direction of the axis of revolution of the latter, using a movable stop applied against the said surface along a generatrix, the said tablet furthermore being immobilized against a fixed stop also applied against the peripheral surface of the tablet along a generatrix diametrically opposite to that against which the movable stop is applied.

9. Pulverulent maltitol, which may be used in particular for the manufacture of a chewing-gum of improved organoleptic quality, characterized in that its maltitol purity is greater than 95% and in that its particle size is such that at least 50% in number of the particles are smaller in size, expressed in microns, than 1.5 times the compressibility value determined in a test A consisting in measuring the force, expressed in newtons, necessary to crush a tablet prepared from the said maltitol, that is to say to cause the appearance of rupture lines within the bulk constituting the latter, this force thus reflecting the crush strength of the tablet, which is cylindrical with plane faces having a diameter of 13 mm, a thickness of 5 mm and a weight of 0.896 g, that is to say having an apparent voluminal mass of 1.35 g/ml, the said force being exerted against the peripheral surface of the tablet in the direction of the axis of revolution of the latter, using a movable stop applied against the said surface along a generatrix, the said tablet furthermore being immobilized against a fixed stop also applied against the peripheral surface of the tablet along a generatrix diametrically opposite to that against which the movable stop is applied.

## Patentansprüche

1. Kaugummi-Zusammensetzung, die eine Gummibase, ein Aroma und pulverförmigen Maltit enthalt, dadurch gekennzeichnet, daß der Maltit eine Reinheit an Maltit größer als 95% und eine solche granulometrische Verteilung aufweist, daß wenigstens 50% der im Inneren der Zusammensetzung vorhandenen Maltit-Teilchen kleiner als 90 Mikrometer sind.

2. Kaugummi-Zusammensetzung gemäß Anspruch 1, dadurch gekennzeichnet, daß der Maltit eine solche granulometrische Verteilung aufweist, daß wenigstens 50% der Teilchen kleiner als 75 Mikrometer, vorzugsweise kleiner als 60 Mikrometer und noch mehr bevorzugt kleiner als 40 Mikrometer sind.

3. Kaugummi-Zusammensetzung gemäß Anspruch 1, dadurch gekennzeichnet, daß der Maltit eine Reinheit an Maltit größer als 98%, vorzugsweise größer als 99% und noch mehr bevorzugt größer als 99,5% aufweist.

4. Kaugummi-Zusammensetzung gemäß Anspruch 1, dadurch gekennzeichnet, daß der Maltit etwa 2 bis etwa 85% der Zusammensetzung, vorzugsweise 5% bis 75% der Zusammensetzung und noch mehr bevorzugt 10% bis 75% der Zusammensetzung darstellt.

5. Kaugummi-Zusammensetzung gemäß Anspruch 1, in der das aromatisierende Mittel etwa 0,2% bis etwa 3%, vorzugsweise 0,5% bis 1,8% und noch mehr bevorzugt 0,8% bis 1,5% der Zusammensetzung darstellt.

6. Kaugummi-Zusammensetzung gemäß Anspruch 1, die außerdem ein Matt-Pulver mit einer Reinheit kleiner als 95% umfaßt.

7. Verfahren, das es erlaubt, die organoleptische Qualität eines Kaugummis bezüglich Geschmack und Textur zu verbessern, dadurch gekennzeichnet, daß es darin besteht, einen pulverförmigen Maltit mit einer Reinheit an Maltit größer als 95% und mit einer Granulometrie und einer Kompressibilität, die so gewählt sind, daß wenigstens 50% der im Innern des Kaugummis vorhandenen Maltit-Teilchen kleiner als 90 Mikrometer sind, zu verwenden.

8. Verwendung, um die organoleptische Qualität eines Kaugummis zu verbessern, eines pulverförmigen Maltits, der eine Reinheit an Maltit größer als 95% und eine solche Granulometrie aufweist, daß wenigstens 50% der Anzahl der Teilchen eine Größe, ausgedrückt in Mikrometern, haben, die kleiner ist als das 1,5-fache des Werts der Kompressibilität des Maltits, bestimmt in einem Test A, der darin besteht, die Kraft, ausgedrückt in Newton, zu messen, die notwendig ist, um das Zerquetschen einer aus dem Maltit hergestellten Tablette zu bewirken, d.h. um das Erscheinen von Bruchlinien im Inneren der Grundmasse derselben zu bewirken, wobei diese Kraft folglich die Beständigkeit ausdrückt gegen das Zerquetschen der Tablette, die zylindrisch ist mit ebenen Flächen mit einem Durchmesser von 13 mm, einer Dicke von 5 mm und einem Gewicht von 0,896 g, d.h. mit einer scheinbaren Dichte von 1,35 g/ml, wobei die Kraft gegen die periphere Oberfläche der Tablette in Richtung der Drehachse derselben ausgeübt wird mittels eines beweglichen Anschlags, der gegen die Oberfläche längs einer Mantellinie angesetzt wird, wobei die Tablette außerdem gegen einen festen Anschlag immobilisiert ist, der ebenfalls gegen die periphere Oberfläche der Tablette längs einer Mantellinie, die derjenigen, gegen die der mobile Anschlag angesetzt wird, diametral gegenüber liegt, angesetzt wird.

9. Pulverförmiger Maltit, der insbesondere für die Herstellung eines Kaugummis, der eine verbesserte organoleptische Qualität besitzt, verwendbar ist, dadurch gekennzeichnet, daß seine Reinheit an Maltit größer als 95% ist, und dadurch, daß seine Granulometrie so ist, daß wenigstens 50% der Anzahl der Teilchen eine Größe, ausgedrückt in Mikrometern, haben, die kleiner ist als das 1,5-fache des Werts der Kompressibilität, bestimmt in einem Test A, der darin besteht, die Kraft, ausgedrückt in Newton, zu messen, die notwendig ist, um das Zerquetschen einer aus dem Maltit hergestellten Tablette zu bewirken, d.h. um das Erscheinen von Bruchlinien im Inneren der Grundmasse derselben zu bewirken, wobei diese Kraft folglich die Beständigkeit ausdrückt gegen das Zerquetschen der Tablette, die zylindrisch ist mit ebenen Flächen mit einem Durchmesser von 13 mm, einer Dicke von 5 mm und einem Gewicht von 0,896 g, d.h. mit einer scheinbaren Dichte von 1,35 g/ml, wobei die Kraft gegen die periphere Oberfläche der Tablette in Richtung der Drehachse derselben ausgeübt wird mittels eines beweglichen Anschlags, der gegen die Oberfläche längs einer Mantellinie angesetzt wird, wobei die Tablette außerdem gegen einen festen Anschlag immobilisiert ist, der ebenfalls gegen die periphere Oberfläche der Tablette längs einer Mantellinie, die derjenigen, gegen die der mobile Anschlag angesetzt wird, diametral gegenüber liegt, angesetzt wird.
